# FASCICULE DE BREVET EUROPEEN

(11) **EP 1 336 398 B1**
(45) Date de publication et mention de la délivrance du brevet: **09.08.2006**
(21) Numéro de dépôt: 02100107.8
(22) Date de dépôt: 05.02.2002
(51) Int. Cl.: A61F 13/26, A61F 13/28, A61F 6/12

(54) **Applicateur pour tampon hygiénique**
Tamponapplikator
Applicator for tampons

(43) Date de publication de la demande: 20.08.2003
(73) Titulaire: BirchBob International SA, 1310 La Hulpe (BE)
(72) Inventeur: Arnould, Marc, 1380 Ohain (BE)
(74) Mandataire: Van Straaten, Joop

(56) Documents cités:
- EP-A- 0 104 039
- EP-A- 0 349 222
- WO-A-83/01741
- FR-A- 2 002 723
- FR-A- 2 546 399
- GB-A- 2 153 684
- US-A- 4 921 474
- US-A- 5 676 647
- US-A- 5 928 183

## Description

L'invention concerne un applicateur pour l'introduction d'un objet solide dans un orifice naturel du corps humain.

L'invention concerne plus particulièrement les tampons hygiéniques. Son principe s'applique également à l'introduction et la mise en place d'autres objets ou appareillages du domaine médical tels que sondes, cathéters, micro caméras, etc.

Un des inconvénients majeurs des tampons hygiéniques est l'inconfort que suscite leur mise en place.

Quoique la mise au point d'un applicateur formé d'un tube et d'un poussoir (connu dans EP 0 551 758) ait constitué une avancée dans le domaine, il n'en reste pas moins beaucoup à faire pour rendre cette opération rapide et indolore.

Un des problèmes réside dans le frottement de l'objet tel qu'un tampon après qu'il soit sorti de l'applicateur, surtout durant les menstruations, les muqueuses étant à ce moment fort sensibles. De nombreux fabricants ont tenté de développer des solutions impliquant l'intégration de produits lubrifiants tels que des gels, soit directement dans les tampons (DE 3739163, GB 2 056 083), soit dans des cavités ou des capsules incorporées dans les applicateurs (US 3,139,886; US 4,421,504).

Un des inconvénients des lubrifiants est qu'ils peuvent limiter les capacités d'absorption des tampons. Par ailleurs, ces lubrifiants donnant, au toucher, une impression graisseuse doivent être libérés in situ, faute de quoi l'applicateur est peu agréable à manipuler.

Une autre approche consiste à arrondir autant que possible la tête de l'applicateur. Dans ce but, certains applicateurs présentent une extrémité distale en forme d'ogive, formée de découpes en pétale qui s'ouvrent vers l'extérieur au passage du tampon (voir EP 0 551 758). Un problème connu est que ces pétales peuvent pincer la chair lors du retrait de l'applicateur.

On a donc cherché à développer un applicateur facile à insérer et qui diminue le frottement de l'objet tel qu'un tampon contre les parois de l'orifice.

L'objet de l'invention est un applicateur pour l'insertion d'un tampon hygiénique dans un orifice naturel du corps féminin comprenant le dit tampon hygiénique inséré dans une enveloppe tubulaire et un poussoir destiné à faire sortir le dit objet de l'enveloppe tubulaire . Cet applicateur comprend un manchon souple fixé concentriquement à l'enveloppe, entre l'extrémité distale de cette enveloppe et le tampon. Le manchon souple qui présente une longueur déployée correspondant sensiblement à celle du tampon se déploie autour du tampon lorsque celui-ci sort de l'enveloppe sous l'effet du poussoir. Le poussoir présente une longueur supérieure à la somme des longueurs de l'enveloppe et du manchon déployé suffisante pour maintenir le tampon en place relativement aux parois de l'orifice naturel lorsqu'on dégage l'objet du manchon en faisant coulisser l'enveloppe et le manchon dans le sens contraire à celui de l'insertion.

Le manchon souple peut être fixé avantageusement à la partie distale de l'enveloppe, à l'extérieur de la partie distale de l'enveloppe ou l'intérieur de l'enveloppe.

Suivant un mode de réalisation avantageux, le manchon souple comprend une partie disposée le long de la paroi extérieure de l'enveloppe.

Le manchon souple est, suivant un mode de réalisation avantageux, initialement ouvert à son extrémité distale. Suivant un autre mode de réalisation avantageux le manchon souple est initialement fermé à son extrémité distale et comprend à cette extrémité une partie destinée à se rompre facilement.

Le matériau du manchon souple présente de préférence un faible coefficient de frottement. Sa face extérieure peut aussi être enduite au moins sur une partie de sa longueur d'une substance lubrifiante. Dans ce cas, suivant un mode de réalisation avantageux, la partie distale du tampon est enduite d'une substance anti-absorbante.

Suivant un mode de réalisation préféré le poussoir comprend deux parties télescopiques et un dispositif de verrouillage de ces deux parties entre elles.

Suivant un mode de réalisation avantageux le poussoir comprend des repères tactiles, ce qui permet une évaluation plus précise de sa position avant le retrait.

Un avantage de l'invention est de permettre d'introduire dans le corps des objets aux formes ou aux contours relativement agressifs avec un minimum d'inconfort. Cet avantage est particulièrement marqué dans le cas de l'introduction de tampons hygiéniques durant les périodes de menstruation, qui se fait sans douleur.

Un autre avantage de l'invention réside dans la possibilité de rectifier la position de l'objet avant le largage.

D'autres particularités et avantages de l'invention ressortiront de la description ci-après de modes de réalisation particuliers de l'invention, référence étant faite aux dessins annexés dans lesquels :
La Fig. 1 est une vue en coupe axiale d'une première forme d'un applicateur suivant l'invention dans le cas d'un tampon hygiénique.
La Fig. 2 est une vue schématique en coupe de l'applicateur de la Fig. 1 après armement et positionnement.
La Fig. 3 est une vue schématique en coupe de l'applicateur de la Fig. 2 en phase de mise en place du tampon.
La Fig. 4 est une vue schématique en coupe de l'applicateur de la Fig. 2 avec le tampon en place.
La Fig. 5 est une vue schématique en coupe de l'applicateur de la Fig. 2 en phase de retrait de l'enveloppe et du manchon.
La Fig. 6 est une vue en coupe axiale d'une autre forme de réalisation de l'invention avec poussoir télescopique.
La Fig. 7 est une vue schématique d'une autre forme de réalisation de l'invention.

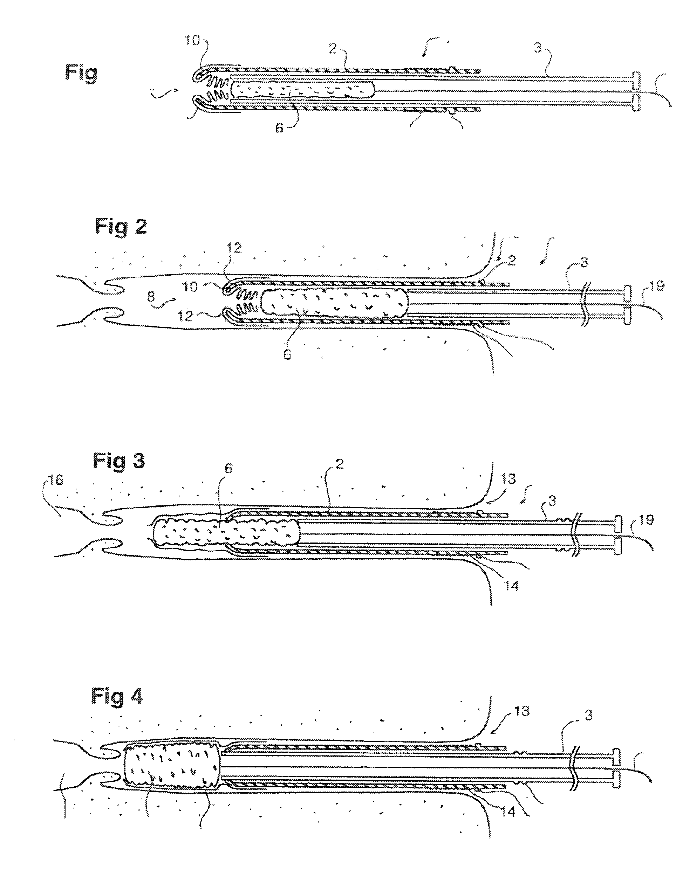

L'applicateur 1 de la Fig. 1, qui est illustré ici dans son rôle d'applicateur 1 de tampon hygiénique, possède en commun avec un applicateur classique une enveloppe tubulaire 2 et un poussoir 3, représenté ici en position de repos, entourant l'objet à insérer, soit un tampon 6. L'enveloppe affecte à sa partie distale 8 une forme en ogive obtenue par une découpe en pétales 10.

À la différence d'un applicateur classique, le présent applicateur comprend un manchon 12 formé d'une membrane souple à faible coefficient de frottement fixé concentriquement à l'enveloppe 2. Ce manchon 12 est fixé ici à l'extérieur de l'enveloppe tubulaire 2, recouvrant son extrémité distale 8, puis est replié à l'intérieur de l'enveloppe 2, entre l'extrémité distale 8 et le tampon 6. Le rôle du manchon 12 sera décrit en se référant aux Figures 2 à 4.

La Fig. 2 représente l'applicateur 1 après que le poussoir 3 ait été armé (c'est-à-dire tiré vers l'arrière et dégagé du tampon 6) et que l'applicateur 1 ait été mis en place dans l'orifice ad hoc 13, en l'occurrence le vagin 13.

Des repères tactiles 14 permettent, en conjonction avec une garde 15, de positionner l'enveloppe tubulaire 2 à la profondeur appropriée. Il est en effet impératif que le tampon 6 ne pénètre pas trop loin et par exemple reste en deçà de la matrice 16.

La Fig. 3 montre le poussoir 3 en phase d'enfoncement : le tampon 6, pressé contre la partie distale 8 de l'enveloppe tubulaire 2, écarte les découpes en pétales 10 et force le manchon 12 à se déployer vers l'extérieur. La membrane du manchon 12 présentant un faible coefficient de frottement se déploie dans le vagin 13, entourant le tampon 6 au fur et à mesure de sa progression, qui se fait avec très peu d'effort.

A la Fig. 4, le tampon 6 est sorti de l'enveloppe 2 et occupe sa position terminale. On constate que la longueur du manchon 12 correspond à celle du tampon 6. Ce dernier étant toujours entouré par le manchon 12, est toujours solidaire de l'applicateur 1, du fait de la faible adhérence du manchon 12 sur la paroi du vagin 14. Il est donc possible, à ce stade, de rectifier légèrement la position du tampon 6(ce qui n'est pas le cas avec un applicateur classique). La présence de seconds repères tactiles 17, ménagés sur le poussoir, permet de vérifier que la manoeuvre a été effectuée de façon correcte.

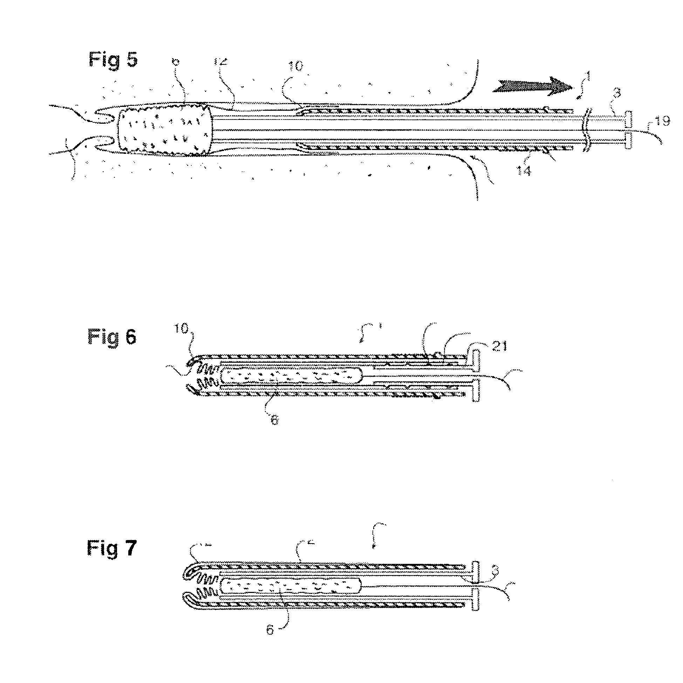

La Fig. 5 illustre une phase supplémentaire propre à l'invention, à savoir le dégagement du tampon par rapport au manchon 12 : on fait coulisser l'enveloppe tubulaire 2 et le manchon 12 le long du poussoir 3 par un mouvement vers l'arrière cependant que le tampon 6 est maintenu bien en place par le poussoir 3. On notera que ce dernier est plus long que dans un applicateur classique. L'ensemble [poussoir 3 + enveloppe 2 et manchon 12] peut être retiré sans problème après dégagement total du tampon 6.

Le manchon 12 est représenté aux Fig. 1 à 4 sous la forme d'un boyau ouvert à ses deux extrémités. Son extrémité proximale étant solidarisée à l'enveloppe 2, il va de soi que l'on peut également prévoir un manchon initialement "fermé" à son extrémité distale, soit par une calotte présentant des lignes d'affaiblissement, soit par un plissage maintenu par un ou plusieurs points de soudures facilement déchirables pour permettre le passage du tampon 6.

Le matériau utilisé pour la fabrication du manchon 12 peut être extrêmement mince, de l'ordre de celui utilisé pour des préservatifs. Le manchon doit présenter une bonne souplesse et glisser facilement, ce qui se traduit par un maximum de douceur sur la paroi vaginale, mais il n'est pas nécessaire qu'il présente par ailleurs des qualités mécaniques exceptionnelles. En effet, il suffit que son diamètre soit légèrement supérieur à celui du tampon 6 pour permettre un glissement sans efforts et sans à-coups. On peut choisir un matériau présentant intrinsèquement un faible coefficient de frottement sur la paroi du vagin 14 ou opter pour un matériau enduit sur tout ou partie de sa surface externe d'un lubrifiant connu par ailleurs. Il a été vérifié expérimentalement que le contact du tampon 6 avec la surface d'un manchon 12 enduite de lubrifiant n'altère virtuellement pas les qualités d'absorption du tampon 6. Le contact ne se fait de toute façon que sur la partie distale du tampon 6. Dans certains cas, on peut toutefois prévoir d'imbiber la pointe du tampon 6 avec un produit neutralisant tout effet d'interaction.

Il ressort clairement de la Fig. 4 que le poussoir 3 du présent applicateur 1 est plus long que le poussoir d'un applicateur normal. Il doit en effet avoir une longueur supérieure à la somme des longueurs de l'enveloppe tubulaire 2 et du manchon 12 déployé et permettre le dégagement décrit à la Fig.5. En jouant sur les longueurs relatives des différents éléments, on constate cependant que l'allongement par rapport à un applicateur plastique classique n'est guère que de 5,5 cm, et de quelques mm par rapport à un applicateur en carton glacé. La longueur du cordon de retrait 19 du tampon 6 est évidemment adaptée à cette particularité.

Un surcroît d'encombrement pouvant nuire au caractère discret de l'applicateur 1 en ce qui concerne son emploi pour des tampons hygiéniques 6 on a cherché à réaliser un modèle particulièrement compact. La Fig. 6 montre un applicateur muni d'un poussoir 3 télescopique. Des butées 18 ou un verrouillage quart de tour permettent de verrouiller les deux éléments 20, 21 du poussoir 3 au moment de l'armement de l'applicateur 1. On a également représenté à la Fig. 6 une autre possibilité de fixation du manchon 12, qui est ici collé à l'intérieur des parois de l'enveloppe 2.

La Fig. 7 montre une autre possibilité de réalisation intéressante du présent applicateur 1 : le manchon 12 est ici collé ou soudé sur l'ensemble de l'enveloppe 2, ce qui permet d'utiliser pour l'enveloppe 2 un matériau présentant un moindre degré de finition tout en renforçant encore le confort à l'introduction. La présence éventuelle d'un lubrifiant est dans ce cas limitée à la partie repliée du manchon 12.

On remarquera que la présence du manchon 12, qu'il soit fixé à l'intérieur ou à l'extérieur de l'enveloppe 2, permet d'éviter tout pincement de la chair par les éventuelles découpes en pétales 10.

Bien que l'applicateur 1 ait été décrit dans le cadre particulier de la mise en place d'un tampon hygiénique, il va de soi que son principe s'applique également à l'introduction et la mise en place d'autres objets ou appareillages du domaine médical tels que sondes, cathéters, micro caméras, certains types de médicaments, etc., et en général tout type d'éléments pouvant s'avérer agressifs pour la paroi d'un organe.

## Revendications

1. Un applicateur (1) pour l'insertion d' un tampon hygiénique (6) dans un orifice naturel (14) du corps féminin comprenant le dit tampon hygiénique (6) inséré dans une enveloppe tubulaire (2) un poussoir (3) destiné à faire sortir le dit tampon hygiénique (6) de l'enveloppe tubulaire (2) et un manchon (12) souple fixé concentriquement à l'enveloppe (2), entre l'extrémité distale (8) de cette enveloppe (2) et le tampon hygiénique (6) **caractérisé en ce que**
- ledit manchon souple (12) se déploie autour du tampon hygiénique (6) lorsque celui-ci sort de l'enveloppe (2) sous l'effet du poussoir (3), la longueur du manchon (12) déployé correspondant sensiblement à celle du tampon hygiénique (6)
- le poussoir (3) ayant une longueur supérieure à la somme des longueurs de l'enveloppe tubulaire (2) et du manchon (12) déployé suffisante pour maintenir le tampon hygiénique (6) en place relativement aux parois de l'orifice naturel (14) lorsqu'on dégage le tampon hygiénique (6) du manchon (12) en faisant coulisser l'enveloppe et le manchon (12) dans le sens contraire à celui de l'insertion.

2. Un applicateur (1) suivant la revendication 1 **caractérisé en ce que** le manchon souple (12) est fixé à la partie distale (8) de l'enveloppe (2).

3. Un applicateur (1) suivant la revendication 2 **caractérisé en ce que** le manchon souple (12) est fixé à l'extérieur de la partie distale (8) de l'enveloppe (2).

4. Un applicateur (1) suivant la revendication 2 **caractérisé en ce que** le manchon souple (12) est fixé à l'intérieur de l'enveloppe (2).

5. Un applicateur (1) suivant la revendication 1 **caractérisé en ce que** le manchon souple (12) comprend une partie disposée le long de la paroi extérieure de l'enveloppe (2).

6. Un applicateur (1) suivant l'une quelconque des revendications précédentes **caractérisé en ce que** le manchon souple (12) est initialement ouvert à son extrémité distale.

7. Un applicateur (1) suivant l'une quelconque des revendications 1 à 5 **caractérisé en ce que** le manchon souple (12) est initialement fermé à son extrémité distale et comprend à cette extrémité une partie destinée à se rompre facilement.

8. Un applicateur (1) suivant l'une quelconque des revendications précédentes **caractérisé en ce que** le matériau du manchon souple (12) présente un faible coefficient de frottement.

9. Un applicateur (1) suivant l'une quelconque des revendications précédentes **caractérisé en ce que** la face extérieure du manchon souple (12) est enduit au moins sur une partie de sa longueur d'une substance lubrifiante.

10. Un applicateur (1) suivant l'une quelconque des revendications 1 à 8 **caractérisé en ce que** la partie distale du tampon (6) est enduite d'une substance anti-absorbante.

11. Un applicateur (1) suivant l'une quelconque des revendications précédentes **caractérisé en ce que** le poussoir (3) comprend deux parties télescopiques (20, 21) et un dispositif de verrouillage (18) de ces deux parties entre elles.

12. Un applicateur (1) suivant l'une quelconque des revendications précédentes **caractérisé en ce que** le poussoir (3) comprend des repères tactiles

## Claims

1. Applicator (1) for inserting a tampon (6) into a natural orifice (14) of a women's body, comprising the said tampon (6) inserted into a tubular outer (2), a plunger (3) intended to expel the said tampon (6) from the tubular outer (2), and a flexible sleeve (12) fixed concentrically to the outer (2), between the distal end (8) of this outer (2) and the tampon (6) **characterized in that**
- the said flexible sleeve (12) deploys around the tampon (6) when the latter leaves the outer (2) under the effect of the plunger (3), the length of the deployed sleeve corresponding substantially to that of the tampon (6)
- the length of the plunger corresponds at least to the sum of the length of the tubular outer and of the deployed flexible sleeve,
the plunger (3) being long enough to hold the tampon (6) in place relative to the wall of the natural orifice (14) while the tampon (6) is disengaged from the sleeve (12) by sliding the outer and the sleeve (12) in the opposite direction to the direction of insertion.

2. Applicator (1) according to claim 1, **characterized in that** the flexible sleeve (12) is fixed to the distal part (8) of the outer (2).

3. Applicator (1) according to Claim 2, **characterized in that** the flexible sleeve (12) is fixed on the outside of the distal part (8) of the outer (2).

4. Applicator (1) according to Claim 2, **characterized in that** the flexible sleeve (12) is fixed on the inside of the outer (2).

5. Applicator (1) according to Claim 1, **characterized in that** the flexible sleeve (12) comprises a part arranged along the exterior wall of the outer (2).

6. Applicator (1) according to any one of the preceding claims, **characterized in that** the flexible sleeve (12) is initially open at its distal end.

7. Applicator (1) according to any one of Claims 1 to 5, **characterized in that** the flexible sleeve (12) is initially closed at its distal end and at that end comprises a part which is intended to rupture easily.

8. Applicator (1) according to any one of the preceding claims, **characterized in that** the material of the flexible sleeve (12) has a low coefficient of friction.

9. Applicator (1) according to any one of the preceding claims, **characterized in that** the exterior face of the flexible sleeve (12) is coated over at least part of its length with a lubricating substance.

10. Applicator (1) according to any one of Claims 1 to 8, **characterized in that** the distal part of the tampon (6) is coated with an anti-absorbent substance.

11. Applicator (1) according to any one of the preceding claims, **characterized in that** the plunger (3) comprises two telescopic parts (20, 21) and a device (18) for locking these two parts together.

12. Applicator (1) according to any one of the preceding claims, **characterized in that** the plunger (3) comprises tactile marks.

## Patentansprüche

1. Applikator (1) zum Einsetzen eines Tampons (6) in eine natürliche Öffnung (14) des weiblichen Körpers, der den Tampon (6), der in eine röhrenförmige Hülle (2) eingesetzt ist, einen Schieber (3), der dazu bestimmt ist, den Tampon (6) aus der röhrenförmigen Hülle (2) austreten zu lassen, und eine geschmeidige Hülse (12), die konzentrisch zwischen dem distalen Ende (8) dieser Hülle (2) und dem Tampon (6) an der Hülle (2) befestigt ist, umfasst, **dadurch gekennzeichnet, dass**
- die geschmeidige Hülse (12) sich um den Tampon (6) entfaltet, wenn dieser unter dem Einfluss des Schiebers (3) aus der Hülle (2) austritt, wobei die Länge der entfalteten Hülse (12) im Wesentlichen derjenigen des Tampons (6) entspricht
- der Schieber (3) eine Länge hat, die die Summe der Längen der röhrenförmigen Hülle (2) und der entfalteten Hülse (12) übertrifft, die ausreicht, um den Tampon (6) in Bezug auf die Wände der natürlichen Öffnung (14) vor Ort zu halten, wenn der Tampon (6) aus der Hülse (12) freigegeben wird, indem die Hülle und die Hülse (12) in die dem Einsetzen entgegen gesetzte Richtung zum Gleiten gebracht werden.

2. Applikator (1) nach Anspruch 1, **dadurch gekennzeichnet, dass** die geschmeidige Hülse (12) am distalen Abschnitt (8) der Hülle (2) befestigt ist.

3. Applikator (1) nach Anspruch 2, **dadurch gekennzeichnet, dass** die geschmeidige Hülse (12) außen am distalen Abschnitt (8) der Hülle (2) befestigt ist.

4. Applikator (1) nach Anspruch 2, **dadurch gekennzeichnet, dass** die geschmeidige Hülse (12) innen an der Hülle (2) befestigt ist.

5. Applikator (1) nach Anspruch 1, **dadurch gekennzeichnet, dass** die geschmeidige Hülse (12) einen Abschnitt umfasst, der entlang der äußeren Wand der Hülle (2) angeordnet ist.

6. Applikator (1) nach einem der vorhergehenden Ansprüche, **dadurch gekennzeichnet, dass** die geschmeidige Hülse (12) anfangs an ihrem distalen Ende geöffnet ist.

7. Applikator (1) nach einem der Ansprüche 1 bis 5, **dadurch gekennzeichnet, dass** die geschmeidige Hülse (12) anfangs an ihrem distalen Ende geschlossen ist und an diesem Ende einen Abschnitt umfasst, der dazu bestimmt ist, leicht zu durchreißen.

8. Applikator (1) nach einem der vorhergehenden Ansprüche, **dadurch gekennzeichnet, dass** das Material der geschmeidigen Hülse (12) einen geringen Reibungskoeffizienten aufweist.

9. Applikator (1) nach einem der vorhergehenden Ansprüche, **dadurch gekennzeichnet, dass** die äußere Seite der geschmeidigen Hülse (12) zumindest auf einem Teil ihrer Länge mit einer Gleitsubstanz überzogen ist.

10. Applikator (1) nach einem der Ansprüche 1 bis 8, **dadurch gekennzeichnet, dass** der distale Abschnitt des Tampons (6) mit einer nicht absorbierenden Substanz überzogen ist.

11. Applikator (1) nach einem der vorhergehenden Ansprüche, **dadurch gekennzeichnet, dass** der Schieber (3) zwei teleskopische Teile (20, 21) und eine Vorrichtung zum Arretieren (18) dieser zwei Teile gegeneinander umfasst.

12. Applikator (1) nach einem der vorhergehenden Ansprüche, **dadurch gekennzeichnet, dass** der Schieber (3) fühlbare Markierungen umfasst.
